# EUROPEAN PATENT SPECIFICATION

(11) **EP 1 993 605 B1**
(45) Date of publication and mention of the grant of the patent: **27.06.2012**
(21) Application number: 07711957.6
(22) Date of filing: 15.03.2007
(51) Int. Cl.: A61K 9/16, A61K 9/00, A61K 35/76, A61K 39/00, A61K 39/39

(54) **DRY POWDER COMPOSITIONS AND SYSTEMS FOR POULTRY VACCINATION**
TROCKENPULVER-ZUSAMMENSETZUNGEN UND SYSTEME ZUR IMPFUNG VON GEFLÜGEL
COMPOSITIONS EN POUDRE SECHE ET SYSTEMES DE VACCINATION DE VOLAILLE

(30) Priority: 15.03.2006 EP 06005245
(43) Date of publication of application: 26.11.2008
(73) Proprietor: Universiteit Gent, 9000 Gent (BE)
(72) Inventor: REMON, Jean, Paul, B-9090 Melle (BE); VERVAET, Chris, B-8870 Izegem (BE); CORBANIE, Evy, B-8790 Waregem (BE); van Eck, Johannes Hubertus Henricus, 3956 VE Leersum (NL); Landman, Wilhelmus Johannes Mathernus, 3581 AD Utrecht (NL)
(74) Representative: Bird, William Edward
(86) International application number: PCT/EP2007/002284
(87) International publication number: WO 2007/104562

(56) References cited:
- WO-A-97/36578
- WO-A-99/52550
- WO-A-2005/084638
- WO-A-2005/123131
- US-B1- 6 651 655
- CORBANIE E A ET AL: "Deposition of differently sized airborne microspheres in the respiratory tract of chickens" AVIAN PATHOLOGY, vol. 35, no. 6, December 2006 (2006-12), page 475, XP009076790 ISSN: 0307-9457
- ALLAN W H ET AL: "A COMPARISON OF THE IMMUNE RESPONSE AND RESPIRATORY STRESS EFFECT OF 3 CLONE SIZES OF VIRUS AND 3 RANGES OF AEROSOL PARTICLE SIZE USING THE LENTOGENIC NEWCASTLE DISEASE VACCINE STRAIN AG-68L" AVIAN PATHOLOGY, vol. 9, no. 2, 1980, pages 153-162, XP009076775 ISSN: 0307-9457
- MESZAROS J ET AL: "IMMUNIZATION OF DAY-OLD CHICKENS AGAINST NEWCASTLE DISEASE" ACTA VETERINARIA HUNGARICA, vol. 40, no. 1-2, 1992, pages 121-127, XP009076774 ISSN: 0236-6290
- ANDYA J D ET AL: "THE EFFECT OF FORMULATION EXCIPIENTS ON PROTEIN STABILITY AND AEROSOL PERFORMANCE OF SPRAY-DRIED POWDERS OF A RECOMBINANT HUMANIZED ANTI-IGE MONOCLONAL ANTIBODY" PHARMACEUTICAL RESEARCH, NEW YORK, NY, US, vol. 16, no. 3, March 1999 (1999-03), pages 350-358, XP008003520 ISSN: 0724-8741

## Description

### Field of the invention

The present invention relates to dry powder compositions for veterinary vaccination useful for inducing protective responses in poultry against avian diseases such as, but not limited to, Newcastle disease and avian influenza. The present invention also relates to systems for performing vaccination in poultry via inhalation by making use of these dry powder compositions.

### Background of the invention

Avian infectious diseases are well known for their negative economic impact on poultry production and, possibly, on the health of other animals (in particular mammals) and human beings which may be directly or indirectly in contact with infected birds.

For example specific avian pathogens such as, but not limited to, Newcastle disease virus (hereinafter abbreviated as NDV), infectious bronchitis virus (IBV), infectious bursal disease virus (IBDV), turkey rhinotracheitis virus (TRTV), infectious laryngotracheitis virus (ILTV), egg drop syndrome (EDS) virus, avian encephalomyelitis virus, reticuloendotheleisis virus, avian pox viruses, avian adenoviruses, infectious coryza, fowl typhoid, fowl cholera, avian influenza, *Mycoplasma gallisepticum, Escherichia coli, Salmonella spp,* etc. may spread from infected poultry.

More specifically, Newcastle disease is a viral infection of poultry with a wide geographical distribution which may cause great economical losses to the poultry industry. NDV is the etiologic agent of said disease and represents the prototype virus of the genus Paramyxovirus. There are nine serotypes of avian paramyxoviruses designated from APMV-I to APMV-9. Newcastle disease is complicated in that different NDV strains may induce very significant variation in the severity of the disease. Infection may take place by either inhalation or ingestion and the infectious form of the virus is then spread from one bird to another.

Avian influenza, or " bird flu ", is a contagious disease of animals caused by Influenza A viruses that normally infect only birds and, less commonly, pigs. Avian influenza viruses tend to be species-specific, but can cross the species barrier to infect mammals and human beings. Influenza A viruses have 16 H subtypes. Only viruses of the H5 and H7 subtypes are known to cause the highly pathogenic form of the disease. The H5N1 virus, responsible for the current worldwide epidemic of bird flu has proved to be especially tenacious, and is presently of particular concern for human health.

Spray and aerosol vaccination, with coarse and fine liquid droplets respectively, are commonly used mass vaccination techniques for the protection of poultry against respiratory viral infections. Methods and devices for nebulization in spray and aerosol vaccination are particularly important for poultry vaccination since it has specific requirements, e.g. the devices have to be operated with an external energy source.

Birds are usually vaccinated a number of times during their lives following the specifications of the manufacturer or tailor made programs in order to optimally prevent disease outbreaks. A so-called primary vaccination (priming) is usually performed in chickens aged below about two to three weeks. This primary vaccination is usually followed by one or more so-called secondary vaccinations (boosters).

Approximately 48 hours after poultry vaccination, vaccination reactions may appear with some respiratory viruses, particularly with NDV. Affected poultry (e.g. chickens) may show respiratory symptoms, anorexia, depression, growth retardation and increased mortality. The vaccination reaction may be further complicated by secondary colibacilosis (*Escherichia coli* infection). In order to prevent this, antibiotic treatments are given to the birds 36 hours after vaccination, which often prove to be efficient. Nevertheless, overuse or misuse of antibiotics may induce bacterial resistance, which may hamper adequate treatment whenever multi-resistant strains aggravate vaccination reactions.

Live viruses of low virulence (lentogenic) or moderate virulence (mesogenic) are commonly used for the vaccination of poultry against Newcastle disease depending on the disease situation. Mesogenic ND vaccine viruses are however suitable only for booster vaccination. However, also in the lentogenic group there is a considerable range in virulence. Commonly used live vaccines include lentogenic strains such as V4, Hitchner B1, F and La Sota, and mesogenic strains such as strain H, Mukteswar, Koinarov and Roakin.

The main advantage of live NDV vaccines is that these can be administered by inexpensive mass application techniques, such as spray, aerosol and drinking water application. However, as mentioned previously, live vaccines may cause severe vaccination reactions, in particular in the lower respiratory tract after aerosol vaccination. In order to minimize this drawback, it is important to use extremely mild virus, especially as agent for primary vaccination. Nevertheless, even the commonly used mild live NDV strains Hitchner B1 and La Sota can still cause moderate respiratory vaccination reactions. Therefore, there is an urgent need in the art for improving the NDV vaccination as to avoid vaccination reactions in inoculated animals, yet producing a solid immunity against the disease.

For aerosol particles to be efficient it is known that inhaled particles should be deposited in specific areas of the respiratory tract of birds taking into account parameters such as, but not limited to, the species of bird, its age, the size of the inhaled particles, and the susceptibility to post-vaccination reactions.

The presently commercially available nebulizers used for the production of coarse sprays generate a significant proportion of particles that will deposit in the deeper airways, i.e. the particle size distributions around the optimal size is too broad. While this effect is desired for booster vaccinations, it may result in adverse vaccination reactions in younger birds.

Newly produced airborne droplets will evaporate to about 10% of their original size. When generated with the currently available spraying techniques, this phenomenon will hamper adequate vaccination results since:
- during primary vaccination, a significant percentage of the vaccine particles will deposit in the deeper airways of birds whereas it should not, thus explaining the high incidence of respiratory post-vaccination reactions observed;
- due to a broad and uncontrolled particle size distribution, only a low percentage of particles evaporated from droplets is inhaled into the lower airways during second vaccination.

Next to an inappropriate droplet size distribution, the loss of vaccine virus due to inactivation during production, reconstitution and nebulization is a second major reason for the low efficiency of known spray and aerosol vaccinations. Firstly, the vaccine virus, which is generally bred in a liquid environment, e.g. the allantoic cavity of specific pathogen-free (SPF) eggs is freeze dried for storage. Although viruses are less susceptible to environmental inactivating factors in dried form and although stabilizing excipients may be added before drying, there is still some loss of virus concentration during live vaccine production and storage. Before nebulization, the vaccine cake needs to be reconstituted and the virus concentration can be further reduced when traces of disinfectant are present in the water used for reconstitution and due to the temperature sensitivity of the virus in a liquid environment. This inactivation increases further when the vaccine droplets evaporate after dispersion. The large reduction in vaccine concentration during spray and aerosol vaccination may endanger the induction of a sufficient immune response. Furthermore, current vaccines in liquid suspensions suffer from a significant lack of stability.

Allan et al. in Avian Pathology (1980) 9:153-162 teaches the use of Newcastle disease virus solutions containing sorbitol dissolved in water to generate, from the surface of a top spinning at very high speeds, wet aerosol particles that are allowed to dry before the exposure chamber by travelling through a series of columns to produce nearly monodisperse aerosols having particle sizes from 1 to 4 µm for chicken vaccination.

WO 97/36578 discloses spray-dried microparticles, at least 90% of which having a volume median particle size of 1 to 10 µm, comprising a substantially uniform mixture of an agent for gene therapy and an excipient such as a carbohydrate. These microparticles may be formulated with a large size carrier and the resulting formulation may be administered using a dry powder inhaler. WO 97/36578 is silent about vaccination, veterinary applications, as well as about microparticle size polydispersity.

WO 99/52550 discloses a polymer particle vaccine delivery system for incorporating a water-insoluble protein antigen, said system being produced from an emulsion of a biodegradable poly(lactide-co-glycolide) copolymer, wherein the resulting polymer particles have an average diameter of 0.05 to 20 µm according to the volume size distribution. Figure 2 shows the size dispersity of exemplary particles having an average diameter of 10 µm. WO 99/52550 is silent about avian virus and nebulization, and does not teach the presence of sugars in the vaccine delivery system.

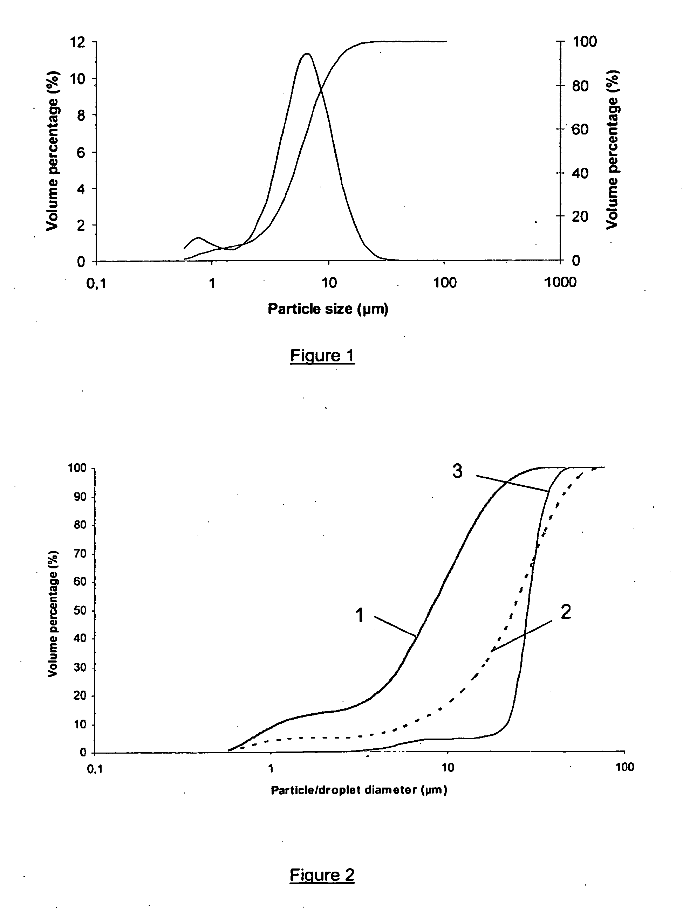

U.S. Patent No. 6,651,655 discloses a method for creating an immune response in a patient using a dry powder inhaler free of any propellant gas, i.e. breath actuated. The vaccine formulation loaded into said dry powder inhaler includes dry powder vaccine particles having an aerodynamic particle size of less than 50 µm, and figure 1 shows the particle size distribution of an exemplary milled vaccine powder. Once such small particles have been produced for deep lung deposition, the micronized substance is blended with large lactose particles, i.e. about 100 µm or about 50 times bigger than the milled vaccine particles. Due to its construction principles, the dry powder inhaler of U.S. Patent No. 6,651,655 is obviously not suitable for poultry vaccination.

WO 2005/123131 discloses formulations for spray-drying an antibody or a vaccine, comprising 4% to 10% by weight of the antibody or a vaccine antigen, 0.1 mM to 50 mM of one or more amino acids, 0.5 to 4% by weight of a sugar, and water. Depending upon the high pressure spraying equipment used, droplets will produce dry powder particles ranging in size from 0.5 to 100 µm. WO 2005/123131 is silent about microparticle size polydispersity and does not consider other formulations than a combination of amino acids and sugars.

Meszaros et al. in Acta Veterinaria Hungaria (1992) 40:121-127 discloses the immunization of 1 to 10 days old chikens against Newcastle disease by aerosol vaccination using a Masterdrop MD-2500 apparatus providing droplets of less than 10 µm droplet size. This publication adds a droplet stabilizer to prevent droplet evaporation to dry particles and is also silent about droplet average size and polydispersity.

The object of the present invention is to overcome these disadvantages.

### Summary of the invention

The herein presented invention enables to overcome the drawbacks of the current poultry vaccination technology in a simple and inexpensive manner by providing efficient dry solid powder compositions for veterinary vaccination, in particular poultry vaccination, which may be used for both primer and booster vaccinations.

Firstly, this invention relates to a dry powder composition for poultry vaccination via inhalation comprising an effective amount of a poultry vaccine agent selected from the group consisting of viruses, bacteria, fungi, parasites and parts thereof, and a supporting amount of carriers for said poultry vaccine agent, said carriers consisting essentially of a combination of a reducing or non-reducing sugar and a biocompatible polymer, wherein said powder composition is in the form of particles having an average particle size from about 2 to 30 µm and a particle size polydispersity from 1.1 to 4.0, and wherein the weight ratio of said biocompatible polymer to said reducing or non-reducing sugar in said combination of carriers is from 1/1 to 1/8. Secondly, the present invention relates to a system for performing vaccinations in poultry via inhalation comprising a chamber containing a dry powder vaccination composition as defined hereinabove, and means for nebulizing the dry powder vaccination composition over said poultry. Thirdly, the present invention relates to methods and processes for producing the above described dry powder poultry vaccination compositions, comprising spray-drying a liquid phase comprising a mixture of a vaccine agent selected from the group consisting of viruses, bacteria, fungi, parasites and parts thereof, a liquid carrier and a supporting amount of solid carriers consisting essentially of a combination of a reducing or non-reducing sugar and a biocompatible polymer to provide said dry powder vaccination composition in the form of particles having an average particle size from 2 to 30 µm.

In one aspect, the invention provides dry powder compositions for poultry vaccination suitable for direct nebulization over poultry without reconstitution of a vaccine solution, therefore overcoming the loss in vaccine concentration inherent to any reconstitution step, and overcoming the significant risk of uncontrolled drying of droplets, which are associated with the current practice in the poultry industry of dissolving a vaccination composition before nebulization over poultry. In this aspect of the present invention, nebulization of a dry powder vaccination composition, without prior reconstitution of a vaccine solution, allows for an easy and efficient distribution and avoids the significant risk of particle agglomeration which may make the particle size unsuitable for inhalation.

### Brief description of the figures

Figure 1 shows the particle size distribution (in microns) of an exemplary dry powder composition for veterinary vaccination according to this invention, in volume percentage both for each fraction (left scale) and for cumulative fractions (right scale).
Figure 2 shows the cumulative particle size distribution of nebulized dry powder vaccines according to the invention, compared to the cumulative droplet size distribution of a nebulized liquid vaccine of the prior art, as measured with laser diffraction technique.
Figure 3 shows the virus concentration expressed as EID₅₀ per m³ of air for a nebulized fine dry powder vaccine according to the invention, compared to a nebulized fine liquid vaccine of the prior art.
Figure 4 shows the average haemagglutination inhibition (HI) serum titres obtained at 2 and 4 weeks post-vaccination with nebulized dry powder vaccines. The error bars represent the standard deviations.

### Detailed description of the invention

The most relevant features of the first aspect of the present invention are:
- the presence of a supporting amount of carriers consisting essentially of a combination of a reducing or non-reducing sugar and a biocompatible polymer, and
- the vaccination powder composition is in the form of particles having an average particle size from 2 to 30 µm, and
- a particle size polydispersity from 1.1 to 4.0, and
- the weight ratio of said biocompatible polymer to said reducing or non-reducing sugar in said combination of carriers is from 1/1 to 1/8

When related to the particle size of a dry vaccination powder, the term " polydispersity " as used herein, unless otherwise stated, refers to the ratio d (84%) / d (50%), i.e. the diameter corresponding to 84% on the cumulative curve of the volume-based particle size distribution divided by the diameter corresponding to 50% on the cumulative curve of the volume-based particle size distribution.

While meeting the objectives of the present invention, useful vaccination compositions may be suitably selected by combining particular sub-ranges of the above stated important parameters, depending upon the type (species) and age of poultry to be vaccinated. In this respect, the type (species) of poultry is not a critical parameter of the invention, and may include not only chickens, but also livestock birds such as turkeys, goose, feasants, ducklings and the like, and pet birds. For instance a powder composition with an average particle size from about 10 to 30 µm, preferably from about 15 to 25 µm, or even better around 20 µm, is especially desired for the first vaccination of chickens aged from one day to about three weeks. On the other hand a powder vaccination composition with an average particle size from about 3 to 9 µm, for instance from about 3 to 6 µm, or around 5 µm, is preferred for the secondary (booster) vaccination of poultry.

A particle size polydispersity as low as possible is an important parameter in the accurate control of the dry powder vaccination compositions and systems of this invention, and is a determining parameter in the final success of the corresponding vaccination methods. Suitable vaccination efficiency can already be obtained while using a dry powder composition of the invention wherein the particle size polydispersity is below 3.0, preferably below 2.5, and even more preferably below 2.0. Since polydispersity control is more difficult to achieve when particle size is lower, and since there should be a fair balance between vaccination efficiency and the costs of polydispersity control, in many instances it may be sufficient for the particle size polydispersity of the dry powder vaccination compositions to be at least 1.3, or at least 1.5, and even at least 1.7 taking into account the conflicting requirements of manufacturing reproducibility and costs, and vaccination efficiency. Suitable working ranges for the particle size polydispersity of the powder composition of this invention are therefore from about 1.3 to about 3.0, preferably from 1.5 to 2.5.

The proportion of the carriers present in the dry powder vaccination composition is not a limiting feature of this invention, and usually does not influence the manufacturing feasibility of the composition. This proportion may be selected by the skilled person, using knowledge standard in the art, depending on various parameters such as, but not limited to, the technical function(s) and number of carriers, their mutual compatibility, the type and concentration of poultry vaccine agent and, optionally, the target average particle size to be achieved (e.g. depending upon the type and age of poultry to be vaccinated). The proportion of said one or more carriers may broadly range from 0.1% to 99.999%, for example from 0.1% to 99.9% such as from 0.5 % to 99.5 % by weight of said dry powder vaccination composition, or from 1 % to 99 %, or from 2 % to 98 %, for instance from 90% to 99.999% by weight. The proportion of the vaccination agent may consequently range from 0.001% to 99.9% by weight, for example, from 0.001% to 10% by weight of said dry powder vaccination composition.

The kind and nature of said one or more carriers present in the dry powder vaccination composition is not a limiting feature of this invention, provided that they participate to vaccine stability and easiness of direct nebulization in powder form, and may be selected by the skilled person, using knowledge standard in the art, depending on various parameters such as, but not limited to, the technical function to be performed by each individual carrier, its compatibility with the other carriers), and cost considerations. Said one or more carriers are preferably of a veterinary acceptable grade and may suitably be selected, but without limitation, from the group consisting of:
- reducing sugars such as, but not limited to, glucose, fructose, mannose, galactose, sorbose, xylose, ribose, lactose, maltose, cellobiose and alike,
- non-reducing sugars such as, but not limited to, trehalose, sucrose, and alike,
- polyols such as, but not limited to, mannitol, sorbitol, maltitol, xylitol, erythritol, inositol and alike,
- biocompatible polymers such as, but not limited to, polyvinylpyrrolidone, polyvinyl acetate, aliphatic polyesters, poly(lactide), poly(glycolide), polylactide-co-glycolide), polycaprolactone, poly(lactide-co-caprolactone), polyacrylic acid, carrageenan, chitosan, cellulose derivatives, dextran, chemically modified dextran, inulin, maltodextrin, polyethylene glycol, polyethylene oxide, polymetacrylates, polyvinyl alcohol, starch and alike,
- proteins such as, but not limited to, albumin, dried milk serum, casein and alike, and
- lipids such as, but not limited to, DPPC, DSPC and alike.

It has surprisingly been found that a combination of a reducing or non-reducing sugar and a biocompatible polymer has superior effects in terms of vaccine stability and easiness of direct nebulization in powder form, over other types of veterinary acceptable carriers known in the art of poultry vaccination. Therefore such a combination constitutes the essential portion of the carriers present in the dry powder vaccination compositions of this invention. In particular circumstances, it may be desirable to complement this specific combination of carriers with other types of carriers specified herein-above such as, but not limited to, polyols, lipids, proteins and the like.

The use of a non-reducing sugar as a carrier in the dry powder vaccination compositions of this invention is usually preferred over reducing sugars. This preference may also be achieved by the use of a mixture thereof, wherein the proportion of non-reducing sugars in the mixture is predominant.

For instance, suitable stabilizing carriers tend to become amorphous upon drying, and/or act as water-replacing materials and/or shield the material by encapsulation, which behaviour may help in preserving the original structure of the microorganism (e.g. virus) contained in the dry powder composition and in contributing to powder stability over time.

Conventional live, attenuated and inactivated vaccines as well as recombinant, subunit, peptide, and DNA vaccines can suitably be used as veterinary vaccination active agent in the powder compositions for poultry vaccination of the invention.

Vaccine agents such as attenuated viruses which may suitably be incorporated into a dry powder composition for poultry vaccination via inhalation according to the present invention can be obtained by any known conventional methods. Briefly, a susceptible substrate may be inoculated with the relevant virus and propagated until the virus replicates to a desired concentration, after which time the virus-containing material is harvested. Any substrate being able to sustain virus replication can suitably be used for performing the present invention, including primary (avian) cell cultures, such as chicken embryo fibroblast cells (CEF) or chicken kidney cells (CK), or mammalian cell lines such as the VERO cell line or baby hamster kidney (BHK) cell line. Preferably, the virus is propagated in specific pathogen free (SPF) chicken embryos.

More particularly, a suitable substrate on which the relevant virus may be propagated is SPF embryonated eggs. Embryonated eggs can be inoculated with, for example 0.2 ml NDV containing allantoic fluid comprising at least 10² embryo infectious dose ₅₀ (herein abbreviated as EID₅₀) per egg. Preferably, 9-12 day-old embryonated eggs are inoculated with about 10⁵ EID₅₀ and subsequently incubated at 37°C for 2 to 4 days, after which time the ND virus product can be harvested, preferably by collecting the allantoic fluid. The fluid can then be centrifuged for about 10 minutes at 2500g, followed by filtering the supernatant through a filter (100 µm).

The present invention also relates to a method of producing the above described dry powder composition for poultry vaccination via inhalation, said method comprising freeze-drying an aqueous formulation comprising a mixture of an effective amount of a poultry vaccination active agent and a supporting amount of a combination of a reducing or non-reducing sugar and a biocompatible polymer, and then milling the direct product of said freeze-drying step such as to provide said powder vaccination composition in the form of dry particles having an average particle size from 2 to 30 µm and, optionally, a particle size polydispersity from 1.1 to 4.0. Sub-ranges of the latter parameters are as described herein-above.

In yet another aspect, the present invention relates to a method of producing the above described dry powder composition for poultry vaccination via inhalation, said method comprising spray-drying a liquid phase comprising a mixture of an effective amount of a veterinary vaccine and a supporting amount of a combination of a reducing or non-reducing sugar and a biocompatible polymer, such as to provide said powder composition in the form of dry particles having an average particle size from 2 µm to 30 µm and, optionally, a particle size polydispersity from about 1.1 to 4.0. The production method of the invention, preferably spray-drying, may be effected at a temperature, i.e. an inlet temperature as explained below, ranging from about 30°C to about 170°C, or even as high as 190°C. The skilled person understands that, depending upon the type of spray-drying equipment and feed rate used, the spray-drying temperature may be quite different at the inlet and the outlet of the spray-drying device. The spray-drying temperature is not a limiting feature of this aspect of the invention, and conventional temperature ranges are from about 40°C to about 160°C. The spray-drying equipment used in this aspect of the invention is not a critical feature of the invention and may be of any type commercially available or standard in the art.

The veterinary vaccination dry powder composition of the present invention is, in view of its average particle size and/or particle size polydispersity characteristics, inhalable by birds and can therefore be efficiently used for either prophylatic or therapeutic immunisation of poultry, e.g. any type of gallinaceous poultry, and a suitable administration route includes, but is not limited to, nebulization of the dry powder vaccination composition over said poultry.

In another aspect, the invention provides dry powder compositions for poultry vaccination suitable for inclusion in a system for direct nebulization over poultry without reconstitution of a vaccine solution, avoiding loss of vaccine concentration and avoiding particle agglomeration.

A further aspect of the invention thus relates to a vaccination system such as a nebulizing equipment including a dry powder composition for poultry vaccination according to the above description. More specifically, this invention relates to a system for performing vaccination in poultry via inhalation comprising :
- a chamber containing a dry powder vaccination composition as defined hereinabove, and
- means for nebulizing the dry powder vaccination composition over said poultry.

Preferably this equipment includes means for pulsing said dry powder composition together with a gas towards poultry. Preferably said gas is air. There is no need for an efficient performance of the invention to use special propellants gases which could be inhaled by the birds and cause problems into the respiratory tract.

The latter aspect of the invention is useful for performing vaccination in poultry by nebulizing a dry powder composition such as described above over said poultry. When this vaccination step is a primary vaccination (primer) in poultry aged below two to three weeks, said dry powder composition is preferably in the form of particles having an average particle size from about 10 to 30 µm, more preferably from about 15 to 25 µm, or even better around 20 µm. When this vaccination is a secondary vaccination (booster) in poultry aged over 2 to 3 weeks, said dry powder composition is preferably in the form of particles having an average particle size from about 3 to 9 µm, more preferably around 5 µm.

A "poultry vaccination composition" as used herein refers to a composition comprising at least one protein, or fragment thereof, which provokes an immune response that protects a bird from illness when administered in effective amounts to said animal. For use in a vaccination composition according to the present invention, the veterinary active agent may suitably comprise a live attenuated, inactivated or recombinant microorganism such as, but not limited to, viruses, bacteria, mycoplasma or chlamydophyla.

Particularly suitable vaccines are such as live, attenuated or inactivated microorganisms and recombinant, subunit, peptide, and DNA vaccines from:
- viruses such as, but not limited to, Paramyxoviridae (e.g. Newcastle Disease Virus, Pneumoviruses), Orthomyxoviridae (e.g. Influenza virus), Coronaviridae (e.g. Infectious Bronchitis virus, Turkey Coronavirus, Turkey Torovirus), Picornaviridae (e.g. Avian Encephalomyelitis virus, Duck Hepatitis virus, Turkey Hepatitis virus), Reoviridae (e.g. Rotavirus), Birnaviridae (e.g. Infectious Bursal Disease Virus), Retroviridae (e.g. Avian Leukosis/Sarcoma virus), Astroviridae, Parvoviridae (e.g. Goose Parvovirus), Adenoviridae, Herpesviridae (e.g. Infectious Laryngotracheitis virus, Marek's Disease virus), Pox viridae, Hepadnaviridae (e.g. Duck Hepatitis virus, Turkey Hepatitis virus), Circoviridae, Papovaviridae (e.g. Goose Hemorrhagic Polyomavirus), Caliciviridae, Togaviridae, Arteriviridae, Flaviviridae, Bunyaviridae, Rhabdoviridae, Filoviridae, Arenaviridae,
- Bacteria such as, but not limited to, Salmonella, Campylobacter, Escherichia Coli, Pasteurella (Fowl Cholera), Rimerella anatipestifer, Ornithobacterium rhinotracheale, Hemophilus paragallinarum, Bordetella avium (Turkey Coryza), Mycoplasma (in particular *Mycoplasma gallisepticum*), Clostridia, Staphylococcus, Streptococcus, Enterococcus, Spirochetes, Mycobacterium and Chlamydophila (psittacosis, ornithosis),
- Fungi such as, but not limited to, Aspergillus and the like, and
- Parasites such as, but not limited to: Nematodes, Acanthocephalans, Coccidia, Cryptosporidium and Cochlosoma anatis.

As an illustration of a particularly useful embodiment of this invention, the following Newcastle disease virus strains may be used:
- lentogenic vaccines such as, but not limited to, Hitchner-B₁, La Sota, Clone 30, V4, NDW, I2 and F, and
- mesogenic vaccines such as, but not limited to, Roakin, Lukteswar and Komarov.

As another illustration of a particularly useful embodiment of this invention, the following avian influenza strain may be used: H5N1 Asia strain.

According to the present invention, immunisation is obtained by inhalation of the dry powder composition of the invention by poultry. For instance, chickens are exposed to a cloud of the dry powder composition having the selected average particle size and the selected combination of carriers, and the selected particle size polydispersity. The duration of the immunity may depend upon the vaccination programme chosen. One of the most important considerations affecting vaccination programmes is the level of maternal immunity in young chickens, which may vary considerably from farm to farm, batch to batch, and among individual chickens. For this reason, one or more of several strategies may be employed as follows. Either the birds are not vaccinated until 2―4 weeks of age when most of them will be susceptible, or 1-day-old birds are vaccinated by the application of a coarse spray. Revaccination is then carried out 3―4 weeks later.

Spray-drying is able to directly deliver dispersible dry powder particles of a suitable average particle size and a suitable particle size polydispersity without the necessity of milling them after the spray-drying step. In this process, a liquid virus suspension is rapidly transformed into a dried particulate form of suitable average particle size and suitable particle size distribution by atomisation in a hot drying gaseous medium such as, but not limited to, air. A large air-water interfacial area is created, water evaporates rapidly and drying completes in a matter of a few seconds. During the early stage of drying, where the droplet surface remains at 100% of relative humidity, the surface temperature maintains at the wet-bulb temperature that is significantly lower that the hot air temperature. The virus itself will not be exposed to the high temperatures, thus contributing to the desired stability of the resulting powder formulation. When water is evaporated, the air around the solid virus-containing particle has already cooled down due to moisture uptake. Denaturation of the virus may also be avoided through the use of carriers being commonly used for the stabilization of labile materials, such as preferably a combination of reducing sugars or non-reducing sugars and biocompatible polymers, and optionally polyols, proteins or lipids, or mixtures thereof in any suitable proportions, as mentioned herein-above.

It is well known from the skilled person that complete dryness of a powder composition cannot easily be achieved or maintained over time unless special and usually expensive production and/or storage conditions are provided. The term " dry " as used herein therefore does not refer to the stringent condition of a complete absence of moisture. If suitably performed with all standard industrial precautions and using commercially available equipment, the spray drying and freeze-drying production methods described herein-above are able to reproducibly achieve powder compositions with a water content not above about 4% by weight, preferably not above about 3% by weight, and more preferably not above about 2% by weight at the time of production. Such levels of residual moisture content of the powder vaccination composition are well acceptable for including said powder into the vaccination system of the invention, and for performing vaccination. If conveniently stored before actual use (i.e. avoiding excessive storage temperature and/or excessive humidity in the storage room, the water content of such dry powder vaccination compositions does not significantly increase over time, in particular does not exceed about 6% by weight after 12 weeks storage.

Typically, the veterinary vaccine powder composition according to the invention can be administered in a dose of about 10³ to 10⁸ EID₅₀ per animal, preferably in a dose ranging from about 10⁵to 10⁷ EID₅₀.

The present invention will be described in the following examples with respect to a particular working embodiment, but the invention is not limited thereto but only by the claims.

### EXAMPLE 1 - preparation of a dry powder vaccination composition by spray-drying

An aqueous solution containing 4% by weight trehalose and 1% by weight polyvinylpyrollidone as carriers and Newcastle Disease virus (2.10¹² EID₅₀ per gram solids) was spray-dried by making use of a Mobile Minor spray-dryer. A 1 mm two-fluid nozzle was operated at an atomizing air pressure of 1 bar. The inlet temperature was set at 160°C, which resulted, together with a feed rate of 26 ml/minute, in an outlet temperature of 70°C.

The resulting powder was evaluated:
- for water content and water absorption using Karl Fischer titration,
- for thermodynamic properties with differential scanning calorimetry, and
- for particle size distribution using laser scattering (see Figure 1), the equipment used being a Mastersizer S long bench version (from Malvern).

Immediately after production, the water content of the resulting powder was 3.1% by weight, and increased to 5.9% by weight after one week of storage in glass vials closed with silica containing caps at 22°C and 42% relative humidity. The material obtained was completely amorphous with a glass transition temperature of 65°C. The particle size polydispersity (as defined herein above) of the dry powder was 1.8, and the average particle size was 6 µm, as shown in Figure 1.

Immediately after drying, an aliquot of the powder was inoculated in SPF eggs and after incubation, the virus content in the allantoic fluids was determined with a haemagglutination test. This showed that 2.10¹¹ EID₅₀ could be retained per gram of spray dried powder.

### EXAMPLE 2 ― comparative evaluation of virus concentrations in the air after nebulization of a fine dry powder vaccine and a liquid vaccine.

A fine dry vaccination powder having a water content of 3.8% by weight, and with a particle size suitable for secondary vaccination of poultry (i.e. for deposition in the lower airways), was prepared according to the invention from an aqueous solution by making use of a Mobile Minor pilot plant dryer with a 1 mm two-fluid nozzle (inlet temperature 160°C, outlet temperature 70°C, feed rate 25 ml/minute, drying gas rate 80 kg/hour, atomizing air pressure 2 bar). The feed solution submitted to spray-drying contained 3% (w/w) trehalose dihydrate, 1% (w/w) polyvinylpyrrolidone (PVP), 1% (w/w) bovine serum albumin (BSA) in distilled water, to which 10⁷ EID₅₀ Clone 30 vaccine virus was added per ml feed solution.

For comparison purposes, a commercial Clone 30 vaccine was reconstituted in 5 ml distilled water, of which 30 µl was further diluted to 5 ml for nebulization with a Walther Pilot I spray-head which is often used for the experimental application of fine secondary aerosol vaccines.

The particle size distribution of the dry powder vaccine of the invention and the droplet size distribution (at nozzle level) of the nebulized comparative liquid vaccine have been measured by laser diffraction. Results are presented in Figure 2, wherein the curve (1) relates to the fine trehalose-PVP-BSA dry powder vaccine of this invention and the curve (2) relates to the fine comparative liquid aerosol. The particle size polydispersity of the dry powder vaccination composition of this invention was 2.0 and its average particle size was 7 µm. The average droplet size of the comparative liquid aerosol was 24 µm.

1 g of the powder vaccine and 5 ml of the diluted liquid vaccine were nebulized in 1.3 m³ isolators (28°C and 40% RH) and immediately after nebulization, air samples were taken on gelatine filters to evaluate the virus concentration in the air (incubation in 10-day-old embryonated eggs and subsequent haemagglutination assay).

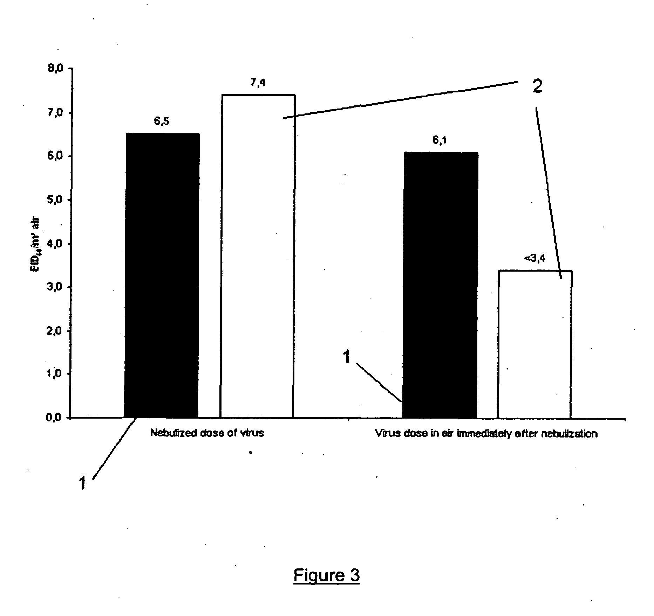

The results are shown in Figure 3, wherein the term " Nebulized dose of virus " represents the theoretical dose of virus that should be present in the air if no virus disappeared from the air by sedimentation or was inactivated by the nebulizing process, and wherein the column (1) relates to the fine trehalose-PVP-BSA dry powder vaccine and the column (2) relates to the comparative nebulized fine liquid vaccine. These results show that high virus concentrations in the air were obtained with the powder vaccine immediately after nebulization, and that the concentration was only slightly lower than the theoretical nebulized concentration (reduction from 10^{6.5} to 10^{6.1}). However, a large reduction of virus titre occurred after nebulization of the fine liquid vaccine (from 10^{7.4} to less than 10^{3.4}), attributed to evaporation of the vaccine droplets at the above mentioned climatic conditions.

### EXAMPLE 3 - Immunization of chickens with a dry powder vaccination composition having an average particle size of 7 µm

The fine dry vaccination powder produced and characterized in example 2 was administered to 4-week-old specified-pathogen-free broiler chickens (i.e. without NCD antibodies), which were housed in isolators with a volume of 1.3 m³. One group received the fine dry powder vaccine, and one group served as non-vaccinated blank control.

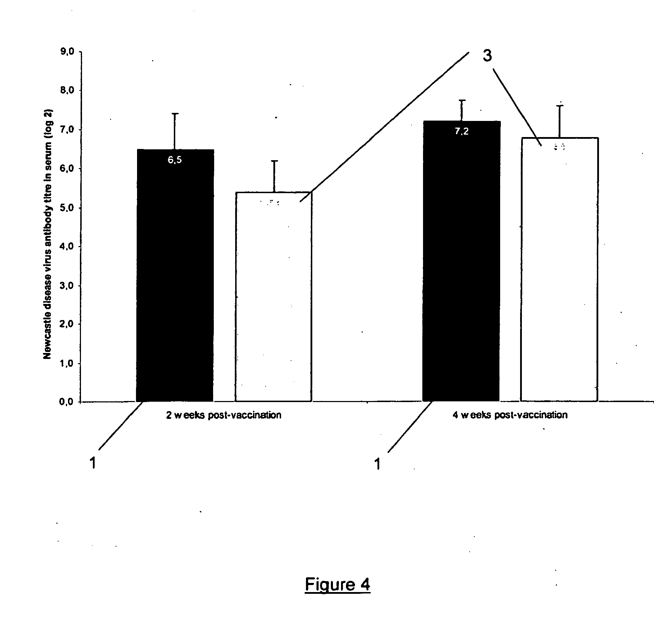

Two and four weeks post-vaccination blood samples were taken and evaluated for antibodies against the NCD virus by haemagglutination inhibition (HI) assay. Results are presented in Figure 4, wherein column (1) relates to the fine trehalose-PVP-BSA powder. The dry power composition of the invention lead to a sufficient immune response at 2 weeks post-vaccination, with HI titres that are higher than the regularly obtained HI titres of 2⁴ to 2⁶ after a single application of a lentogenic NCD vaccine. Statistical analysis by repeated measures ANOVA design, showed a significant increase of titre from 2 to 4 weeks post-vaccination.

### EXAMPLE 4 - Immunization of chickens with a coarse dry powder vaccination composition

A coarse dry vaccination powder having a water content of 3.3% by weight, and with a particle size suitable for primary vaccination (i.e. for deposition in the upper airways) was prepared from the same aqueous feed solution as in example 2 but using a spray dryer with a 30 µm piezo-actuated nozzle (inlet temperature 90°C, outlet temperature 70°C, feed rate 30 ml/minute, drying gas flow rate 25 kg/hour, droplet generation frequency 50 kHz).

The particle size distribution was evaluated by the same method and equipment as in example 2 (laser diffraction) and results are presented in Figure 2, wherein the curve (3) relates to the coarse trehalose-PVP-BSA dry powder vaccine. The particle size polydispersity was 1.2 and the average particle size was 29 µm.

The vaccine was administered to 4-week-old specified-pathogen-free broiler chickens (i.e. without NCD antibodies), which were housed in isolators with a volume of 1.3 m³. One group received the coarse dry powder vaccine, and one group served as non-vaccinated blank control.

Two and four weeks post-vaccination blood samples were taken and evaluated for antibodies against the NCD virus by haemagglutination inhibition (HI) assay. Results are presented in Figure 4, wherein column (3) relates to the coarse trehalose-PVP-BSA powder. The dry power composition of the invention lead to a sufficient immune response at 2 weeks post-vaccination, with HI titres that are within the range of regularly obtained HI titres of 2⁴ to 2⁶ after a single application of a lentogenic NCD vaccine. Statistical analysis by repeated measures ANOVA design, showed a significant increase of titre from 2 to 4 weeks post-vaccination. The significantly lower immune response compared to the fine trehalose-PVP-BSA powder of example 3 relates to the different deposition sites of the vaccine (i.e. upper airways for the coarse powder, lower airways for the fine powder).

## Claims

1. A dry powder composition for poultry vaccination via inhalation comprising an effective amount of a poultry vaccine agent selected from the group consisting of viruses, bacteria, fungi, parasites and parts thereof, and a supporting amount of carriers for said poultry vaccine agent, said carriers consisting essentially of a combination of a reducing or non-reducing sugar and a biocompatible polymer, said dry powder composition being in the form of particles having an average particle size from 2 to 30 µm and a particle size polydispersity from 1.1 to 4.0, and wherein the weight ratio of said biocompatible polymer to said reducing or non-reducing sugar in said combination of carriers is from 1/1 to 1/8.

2. A dry powder composition for poultry vaccination according to claim 1, wherein the virus is selected from the group consisting of Paramyxoviridae, Newcastle Disease Virus, Pneumoviruses, Orthomyxoviridae, Influenza virus, Coronaviridae, Infectious Bronchitis virus, Turkey Coronavirus, Turkey Torovirus, Picornaviridae, Avian Encephalomyelitis virus, Duck Hepatitis virus, Turkey Hepatitis virus, Reoviridae, Rotavirus, Birnaviridae, Infectious Bursal Disease Virus, Retroviridae, Avian Leukosis/Sarcoma virus, Astroviridae, Parvoviridae, Goose Parvovirus, Adenoviridae, Herpesviridae, Infectious Laryngotracheitis virus, Marek's Disease virus, Pox viridae, Hepadnaviridae, Duck Hepatitis virus, Turkey Hepatitis virus, Circoviridae, Papovaviridae, Goose Hemorrhagic Polyomavirus, Caliciviridae, Togaviridae, Arteriviridae, Flaviviridae, Bunyaviridae, Rhabdoviridae, Filoviridae, Arenaviridae,

3. A dry powder composition for poultry vaccination according to claim 1 or claim 2, wherein said dry powder is in the form of particles having an average particle size from 10 to 30 µm.

4. A dry powder composition for poultry vaccination according to claim 1 or claim 2, wherein said dry powder is in the form of particles having an average particle size from 3 to 9 µm.

5. A dry powder composition for poultry vaccination according to any of claims 1 to 4, wherein said reducing sugar is selected from the group consisting of glucose, fructose, mannose, galactose, sorbose, xylose, ribose, lactose, maltose, cellobiose, and mixtures thereof.

6. A dry powder composition for poultry vaccination according to any one of claims 1 to 5, wherein said non-reducing sugar is selected from the group consisting of trehalose and sucrose.

7. A dry powder composition for poultry vaccination according to any one of claims 1 to 6, wherein said biocompatible polymer is selected from the group consisting of polyvinylpyrrolidone, polyvinyl acetate, aliphatic polyesters, poly(lactide), poly(glycolide), polylactide-co-glycolide), polycaprolactone, poly(lactide-co-caprolactone), polyacrylic acid, carrageenan, chitosan, cellulose derivatives, dextran, chemically modified dextran, inulin, maltodextrin, polyethylene glycol, polyethylene oxide, polymetacrylates, polyvinyl alcohol and starch.

8. A method of producing a dry powder vaccination composition according to claim 1, said method comprising spray-drying a liquid phase comprising a mixture of a vaccine agent selected from the group consisting of viruses, bacteria, fungi, parasites and parts thereof, a liquid carrier and a supporting amount of solid carriers consisting essentially of a combination of a reducing or non-reducing sugar and a biocompatible polymer to provide said dry powder vaccination composition in the form of particles having an average particle size from 2 to 30 µm.

9. A method according to claim 8, wherein the virus is selected from the group consisting of Paramyxoviridae, Newcastle Disease Virus, Pneumoviruses, Orthomyxoviridae, Influenza virus, Coronaviridae, Infectious Bronchitis virus, Turkey Coronavirus, Turkey Torovirus, Picornaviridae, Avian Encephalomyelitis virus, Duck Hepatitis virus, Turkey Hepatitis virus, Reoviridae, Rotavirus, Birnaviridae, Infectious Bursal Disease Virus, Retroviridae, Avian Leukosis/Sarcoma virus, Astroviridae, Parvoviridae, Goose Parvovirus, Adenoviridae, Herpesviridae, Infectious Laryngotracheitis virus, Marek's Disease virus, Pox viridae, Hepadnaviridae, Duck Hepatitis virus, Turkey Hepatitis virus, Circoviridae, Papovaviridae, Goose Hemorrhagic Polyomavirus, Caliciviridae, Togaviridae, Arteriviridae, Flaviviridae, Bunyaviridae, Rhabdoviridae, Filoviridae, Arenaviridae,

10. A method according to claim 8 or claim 9, wherein spray-drying is effected at a temperature ranging from 160°C to 190°C.

11. A system for performing vaccination in poultry via inhalation comprising :
- a chamber containing a dry powder vaccination composition according to claim 1, and
- means for nebulizing the dry powder vaccination composition over said poultry.

12. A system for performing vaccination in poultry according to claim 11, wherein the virus is selected from the group consisting of Paramyxoviridae, Newcastle Disease Virus, Pneumoviruses, Orthomyxoviridae, Influenza virus, Coronaviridae, Infectious Bronchitis virus, Turkey Coronavirus, Turkey Torovirus, Picornaviridae, Avian Encephalomyelitis virus, Duck Hepatitis virus, Turkey Hepatitis virus, Reoviridae, Rotavirus, Birnaviridae, Infectious Bursal Disease Virus, Retroviridae, Avian Leukosis/Sarcoma virus, Astroviridae, Parvoviridae, Goose Parvovirus, Adenoviridae, Herpesviridae, Infectious Laryngotracheitis virus, Marek's Disease virus, Pox viridae, Hepadnaviridae, Duck Hepatitis virus, Turkey Hepatitis virus, Circoviridae, Papovaviridae, Goose Hemorrhagic Polyomavirus, Caliciviridae, Togaviridae, Arteriviridae, Flaviviridae, Bunyaviridae, Rhabdoviridae, Filoviridae and Arenaviridae.

13. A system for performing vaccination in poultry according to claim 11 or claim 12, wherein the water content of said dry powder composition is not above 4% by weight.

14. A system for performing vaccination in poultry according to any one of claims 11 to 13, wherein the non-reducing sugar of the dry powder vaccination composition contained in said chamber is selected from the group consisting of trehalose and sucrose.

15. A system for performing vaccination in poultry according to any one of claims 11 to 14, wherein the biocompatible polymer of the dry powder vaccination composition contained in said chamber is selected from the group consisting of polyvinylpyrrolidone, polyvinyl acetate, aliphatic polyesters, poly(lactide), poly(glycolide), polylactide-co-glycolide), polycaprolactone, poly(lactide-co-caprolactone), polyacrylic acid, carrageenan, chitosan, cellulose derivatives, dextran, chemically modified dextran, inulin, maltodextrin, polyethylene glycol, polyethylene oxide, polymetacrylates, polyvinyl alcohol and starch

## Patentansprüche

1. Trockenpulverzusammensetzung zur Geflügelimpfung durch Inhalation, umfassend eine wirksame Menge eines Geflügelimpfstoffs, ausgewählt aus der Gruppe bestehend aus Viren, Bakterien, Pilzen, Parasiten und Teilen davon, und eine unterstützende Menge von Trägern für den Geflügelimpfstoff, wobei die Träger im Wesentlichen aus einer Kombination eines reduzierenden oder nicht reduzierenden Zuckers und eines biokompatiblen Polymers bestehen, wobei die Trockenpulverzusammensetzung die Form von Partikeln mit einer durchschnittlichen Partikelgröße von 2 bis 30 µm und einer Partikelgrößen-Polydispersität von 1,1 bis 4,0 aufweist und wobei das Gewichtsverhältnis des biokompatiblen Polymers zu dem reduzierenden oder nicht reduzierenden Zucker in der Kombination von Trägern von 1/1 bis 1/8 reicht.

2. Trockenpulverzusammensetzung zur Geflügelimpfung nach Anspruch 1, wobei das Virus ausgewählt ist aus der Gruppe bestehend aus Paramyxoviridae, Newcastle-Krankheitsvirus, Pneumoviren, Orthomyxoviridae, Influenzavirus, Coronaviridae, Infektiösem Bronchitisvirus, Truthahn-Coronavirus, Truthahn-Torovirus, Picomaviridae, Vogel-Encephalomyelitisvirus, Enten-Hepatitisvirus, Truthahn-Hepatitisvirus, Reoviridae, Rotavirus, Birnaviridae, Infektiösem Bursa-Krankheitsvirus, Retroviridae, Vogel-Leukose/Sarcomavirus, Astroviridae, Parvoviridae, Gänse-Parvovirus, Adenoviridae, Herpesviridae, Infektiösem Laryngotracheitisvirus, Marek-Krankheitsvirus, Poxviridae, Hepadnaviridae, Enten-Hepatitisvirus, Truthahn-Hepatitisvirus, Circoviridae, Papovaviridae, Gänse-Hämorrhagischem Polyomavirus, Caliciviridae, Togaviridae, Arteriviridae, Flaviviridae, Bunyaviridae, Rhabdoviridae, Filoviridae und Arenaviridae.

3. Trockenpulverzusammensetzung zur Geflügelimpfung nach Anspruch 1 oder Anspruch 2, wobei das Trockenpulver die Form von Partikeln mit einer durchschnittlichen Partikelgröße von 10 bis 30 µm aufweist.

4. Trockenpulverzusammensetzung zur Geflügelimpfung nach Anspruch 1 oder Anspruch 2, wobei das Trockenpulver die Form von Partikeln mit einer durchschnittlichen Partikelgröße von 3 bis 9 µm aufweist.

5. Trockenpulverzusammensetzung zur Geflügelimpfung nach einem der Ansprüche 1 bis 4, wobei der reduzierende Zucker ausgewählt ist aus der Gruppe bestehend aus Glucose, Fructose, Mannose, Galactose, Sorbose, Xylose, Ribose, Lactose, Maltose, Cellobiose und Gemischen davon.

6. Trockenpulverzusammensetzung zur Geflügelimpfung nach einem der Ansprüche 1 bis 5, wobei der nicht reduzierende Zucker ausgewählt ist aus der Gruppe bestehend aus Trehalose und Sucrose.

7. Trockenpulverzusammensetzung zur Geflügelimpfung nach einem der Ansprüche 1 bis 6, wobei das biokompatible Polymer ausgewählt ist aus der Gruppe bestehend aus Polyvinylpyrrolidon, Polyvinylacetat, aliphatischen Polyestern, Poly(lactid), Poly(glycolid), Poly(lactid-co-glycolid), Polycaprolacton, Poly(lactid-co-caprolacton), Polyacrylsäure, Carrageen, Chitosan, Zellulosederivaten, Dextran, chemisch modifiziertem Dextran, Inulin, Maltodextrin, Polyethylenglycol, Polyethylenoxid, Polymethacrylaten, Polyvinylalkohol und Stärke.

8. Verfahren zur Herstellung einer Trockenpulverimpfungszusammensetzung nach Anspruch 1, wobei das Verfahren das Sprühtrocken einer flüssigen Phase umfasst, die ein Gemisch aus einem Impfstoff, der ausgewählt aus der Gruppe bestehend aus Viren, Bakterien, Pilzen, Parasiten und Teilen davon, einem flüssigen Träger und einer unterstützenden Menge von festen Trägern, die im Wesentlichen aus einer Kombination eines reduzierenden oder nicht reduzierenden Zuckers und eines biokompatiblen Polymers bestehen, umfasst, um die Trockenpulverimpfungszusammensetzung in der Form von Partikeln mit einer durchschnittlichen Partikelgröße von 2 bis 30 µm bereitzustellen.

9. Verfahren nach Anspruch 8, wobei das Virus ausgewählt ist aus der Gruppe bestehend aus Paramyxoviridae, Newcastle-Krankheitsvirus, Pneumoviren, Orthomyxoviridae, Influenzavirus, Coronaviridae, Infektiösem Bronchitisvirus, Truthahn-Coronavirus, Truthahn-Torovirus, Picomaviridae, Vogel-Encephalomyelitisvirus, Enten-Hepatitisvirus, Truthahn-Hepatitisvirus, Reoviridae, Rotavirus, Birnaviridae, Infektiösem Bursa-Krankheitsvirus, Retroviridae, Vogel-Leukose/Sarcomavirus, Astroviridae, Parvoviridae, Gänse-Parvovirus, Adenoviridae, Herpesviridae, Infektiösem Laryngotracheitisvirus, Marek-Krankheitsvirus, Poxviridae, Hepadnaviridae, Enten-Hepatitisvirus, Truthahn-Hepatitisvirus, Circoviridae, Papovaviridae, Gänse-Hämorrhagischem Polyomavirus, Caliciviridae, Togaviridae, Arteriviridae, Flaviviridae, Bunyaviridae, Rhabdoviridae, Filoviridae und Arenaviridae.

10. Verfahren nach Anspruch 8 oder Anspruch 9, wobei die Sprühtrocknung bei einer Temperatur im Bereich von 160°C bis 190°C durchgeführt wird.

11. System zur Durchführung einer Impfung bei Geflügel durch Inhalation, umfassend:
- eine Kammer, die eine Trockenpulverimpfungszusammensetzung nach Anspruch 1 enthält, und
- Mittel zum Zerstäuben der Trockenpulverimpfungszusammensetzung über dem Geflügel.

12. System zur Durchführung einer Impfung bei Geflügel nach Anspruch 11, wobei das Virus ausgewählt ist aus der Gruppe bestehend aus Paramyxoviridae, Newcastle-Krankheitsvirus, Pneumoviren, Orthomyxoviridae, Influenzavirus, Coronaviridae, Infektiösem Bronchitisvirus, Truthahn-Coronavirus, Truthahn-Torovirus, Picomaviridae, Vogel-Encephalomyelitisvirus, Enten-Hepatitisvirus, Truthahn-Hepatitisvirus, Reoviridae, Rotavirus, Bimaviridae, Infektiösem Bursa-Krankheitsvirus, Retroviridae, Vogel-Leukose/Sarcomavirus, Astroviridae, Parvoviridae, Gänse-Parvovirus, Adenoviridae, Herpesviridae, Infektiösem Laryngotracheitisvirus, Marek-Krankheitsvirus, Poxviridae, Hepadnaviridae, Enten-Hepatitisvirus, Truthahn-Hepatitisvirus, Circoviridae, Papovaviridae, Gänse-Hämorrhagischem Polyomavirus, Caliciviridae, Togaviridae, Arteriviridae, Flaviviridae, Bunyaviridae, Rhabdoviridae, Filoviridae und Arenaviridae.

13. System zur Durchführung einer Impfung bei Geflügel nach Anspruch 11 oder Anspruch 12, wobei der Wassergehalt der Trockenpulverzusammensetzung nicht über 4 Gewichtsprozent liegt.

14. System zur Durchführung einer Impfung bei Geflügel nach einem der Ansprüche 11 bis 13, wobei der nicht reduzierende Zucker der Trockenpulverimpfungszusammensetzung, die in der Kammer enthalten ist, ausgewählt ist aus der Gruppe bestehend aus Trehalose und Sucrose.

15. System zur Durchführung einer Impfung bei Geflügel nach einem der Ansprüche 11 bis 14, wobei das biokompatible Polymer der Trockenpulverimpfungszusammensetzung, die in der Kammer enthalten ist, ausgewählt ist aus der Gruppe bestehend aus Polyvinylpyrrolidon, Polyvinylacetat, aliphatischen Polyestern, Poly(lactid), Poly(glycolid), Polylactid-co-glycolid), Polycaprolacton, Poly(lactid-co-caprolacton), Polyacrylsäure, Carrageen, Chitosan, Zellulosederivaten, Dextran, chemisch modifiziertem Dextran, Inulin, Maltodextrin, Polyethylenglycol, Polyethylenoxid, Polymethacrylaten, Polyvinylalkohol und Stärke.

## Revendications

1. Composition de poudre sèche pour la vaccination de volaille par inhalation comprenant une quantité efficace d'un agent de vaccin de volaille sélectionné à partir du groupe constitué par des virus, des bactéries, des moisissures, des parasites et des parties de ceux-ci, et une quantité de support de vecteurs pour ledit agent de vaccin de volaille, lesdits vecteurs consistant sensiblement en une combinaison d'un sucre réducteur ou non réducteur et d'un polymère compatible d'un point de vue biologique, ladite composition de poudre sèche étant sous la forme de particules ayant une taille de particule moyenne de 2 à 30 *µ*m et une polydispersité de taille de particule de 1,1 à 4,0, et dans laquelle le rapport pondéral dudit polymère compatible d'un point de vue biologique sur ledit sucre réducteur ou non réducteur dans ladite combinaison de vecteurs est de 1/1 à 1/8.

2. Composition de poudre sèche pour la vaccination de volaille selon la revendication 1, dans laquelle le virus est sélectionné à partir du groupe constitué par les Paramyxovirus, le virus de la maladie de Newcastle, les Pneumovirus, les Orthomyxovirus, le virus de la grippe, les Coronavirus, le virus de la bronchite Infectieuse, le Coronavirus du dindon, le Torovirus du dindon, les Picornavirus, le virus de l'encéphalomyélite aviaire, le virus de l'hépatite du canard, le virus de l'hépatite du dindon, les Réovirus, les Rotavirus, les Birnavirus, le virus de la bursite infectieuse, les Rétrovirus, le virus de la leucose / sarcome aviaire, les Astrovirus, les Parvovirus, le Parvovirus de l'oie, les Adénovirus, les Herpesviridae, le virus de la laryngotrachéite Infectieuse, le virus de la maladie de Marek, les virus du groupe Pox, les Hépadnavirus, le virus de l'hépatite du canard, le virus de l'hépatite du dindon, les Circovirus, les virus du groupe Papova, le Polyomavirus hémorragique de l'oie, les Calicivirus, les Togaviridae, les Artérivirus, les Flaviviridés, les Bunyaviridés, les Rhabdoviridés, les Filovirus, les Arénaviridés.

3. Composition de poudre sèche pour la vaccination de volaille selon la revendication 1 ou la revendication 2, dans laquelle ladite poudre sèche est sous la forme de particules ayant une taille de particule moyenne de 10 à 30 *µ*m.

4. Composition sèche de poudre pour la vaccination de volaille selon la revendication 1 ou la revendication 2, dans laquelle ladite poudre sèche est sous la forme de particules ayant une taille de particule moyenne de 3 à 9 *µ*m.

5. Composition de poudre sèche pour la vaccination de volaille selon l'une quelconque des revendications 1 à 4, dans laquelle ledit sucre réducteur est sélectionné à partir du groupe constitué par le glucose, le fructose, le mannose, le galactose, le sorbose, le xylose, le ribose, le lactose, le maltose, le cellobiose et des mélanges de ceux-ci.

6. Composition de poudre sèche pour la vaccination de volaille selon l'une quelconque des revendications 1 à 5, dans laquelle ledit sucre non réducteur est sélectionné à partir du groupe constitué par le tréhalose et le saccharose.

7. Composition de poudre sèche pour la vaccination de volaille selon l'une quelconque des revendications 1 à 6, dans laquelle ledit polymère compatible d'un point de vue biologique est sélectionné à partir du groupe constitué par le polyvinylpyrrolidone, l'acétate de polyvinyle, des polyesters aliphatiques, le poly(lactide), le poly(glycolide), le poly(lactide-co-glycolide), le polycaprolactone, le poly(lactide-co-caprolactone), l'acide polyacrylique, la carraghénine, le chitosane, les dérivés de cellulose, la dextrane, la dextrane chimiquement modifiée, l'inuline, la maltodextrine, le polyéthylène glycol, l'oxyde de polyéthylène, les polymétacrylates, l'alcool de polyvinyle et l'amidon.

8. Procédé de production d'une composition de poudre sèche pour vaccination selon la revendication 1, ledit procédé comprenant le séchage par atomisation d'une phase liquide comprenant un mélange d'un agent de vaccin sélectionné à partir du groupe constitué par des virus, des bactéries, des moisissures, des parasites et des parties de ceux-ci, un vecteur liquide et une quantité de support de vecteurs solides consistant sensiblement en une combinaison d'un sucre réducteur ou non réducteur et d'un polymère compatible d'un point de vue biologique pour fournir ladite composition de poudre sèche pour vaccination sous la forme de particules ayant une taille de particule moyenne de 2 à 30 *µ*m.

9. Procédé selon la revendication 8, dans lequel le virus est sélectionné à partir du groupe constitué par les Paramyxovirus, le virus de la maladie de Newcastle, les Pneumovirus, les Orthomyxovirus, le virus de la grippe, les Coronavirus, le virus de la bronchite Infectieuse, le Coronavirus du dindon, le Torovirus du dindon, les Picornavirus, le virus de l'encéphalomyélite aviaire, le virus de l'hépatite du canard, le virus de l'hépatite du dindon, les Réovirus, les Rotavirus, les Birnavirus, le virus de la bursite infectieuse, les Rétrovirus, le virus de la leucose / sarcome aviaire, les Astrovirus, les Parvovirus, le Parvovirus de l'oie, les Adénovirus, les Herpesviridae, le virus de la laryngotrachéite Infectieuse, le virus de la maladie de Marek, les virus du groupe Pox, les Hépadnavirus, le virus de l'hépatite du canard, le virus de l'hépatite du dindon, les Circovirus, les virus du groupe Papova, le Polyomavirus hémorragique de l'oie, les Calicivirus, les Togaviridae, les Artérivirus, les Flaviviridés, les Bunyaviridés, les Rhabdoviridés, les Filovirus, les Arénaviridés.

10. Procédé selon la revendication 8 ou la revendication 9, dans lequel le séchage par atomisation est effectué à une température allant de 160 °C à 190 °C.

11. Système pour effectuer la vaccination de volaille par inhalation comprenant :
- une chambre contenant une composition de poudre sèche pour vaccination selon la revendication 1, et
- un moyen pour vaporiser la composition de poudre sèche pour vaccination sur ladite volaille.

12. Système pour effectuer la vaccination de volaille selon la revendication 11, dans lequel le virus est sélectionné à partir du groupe constitué par les Paramyxovirus, le virus de la maladie de Newcastle, les Pneumovirus, les Orthomyxovirus, le virus de la grippe, les Coronavirus, le virus de la bronchite Infectieuse, le Coronavirus du dindon, le Torovirus du dindon, les Picornavirus, le virus de l'encéphalomyélite aviaire, le virus de l'hépatite du canard, le virus de l'hépatite du dindon, les Réovirus, les Rotavirus, les Birnavirus, le virus de la bursite infectieuse, les Rétrovirus, le virus de la leucose / sarcome aviaire, les Astrovirus, les Parvovirus, le Parvovirus de l'oie, les Adénovirus, les Herpesviridae, le virus de la laryngotrachéite Infectieuse, le virus de la maladie de Marek, les virus du groupe Pox, les Hépadnavirus, le virus de l'hépatite du canard, le virus de l'hépatite du dindon, les Circovirus, les virus du groupe Papova, le Polyomavirus hémorragique de l'oie, les Calicivirus, les Togaviridae, les Artérivirus, les Flaviviridés, les Bunyaviridés, les Rhabdoviridés, les Filovirus, les Arénaviridés.

13. Système pour effectuer la vaccination de volaille selon la revendication 11 ou la revendication 12, dans lequel la teneur en eau de ladite composition de poudre sèche n'est pas supérieure à 4 % en poids.

14. Système pour effectuer la vaccination de volaille selon l'une quelconque des revendications 11 à 13, dans lequel le sucre non réducteur de la composition de poudre sèche pour vaccination contenue dans ladite chambre est sélectionné à partir du groupe constitué par le tréhalose et le saccharose.

15. Système pour effectuer la vaccination de volaille selon l'une quelconque des revendications 11 à 14, dans lequel le polymère compatible d'un point de vue biologique de la composition de poudre sèche pour vaccination contenue dans ladite chambre est sélectionné à partir du groupe constitué par le polyvinylpyrrolidone, l'acétate de polyvinyle, des polyesters aliphatiques, le poly(lactide), le poly(glycolide), le poly(lactide-co-glycolide), le polycaprolactone, le poly(lactide-co-caprolactone), l'acide polyacrylique, la carraghénine, le chitosane, les dérivés de cellulose, la dextrane, la dextrane chimiquement modifiée, l'inuline, la maltodextrine, le polyéthylène glycol, l'oxyde de polyéthylène, les polymétacrylates, l'alcool de polyvinyle et l'amidon.
